(19) Europäisches Patentamt
European Patent Office
Office européen des brevets

(11) **EP 1 985 228 A2**

(12) **EUROPEAN PATENT APPLICATION**

(43) Date of publication:
**29.10.2008 Bulletin 2008/44**

(51) Int Cl.:
**A61B 5/00** (2006.01)

(21) Application number: **08250519.9**

(22) Date of filing: **13.02.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **15.02.2007 GB 0702969**

(71) Applicant: **Laerdal Medical AS**
**4002 Stavanger (NO)**

(72) Inventors:
• **Nysaether, Jon**
 **4041 Hafrsfjord (NO)**
• **Eilevstjonn, Joar**
 **4323 Sandnes (NO)**

(74) Representative: **Briddes, Sam**
 **Onsagers Ltd**
 **c/o Innovation Norway**
 **5 Lower Regent Street**
 **London SW1Y 4LR (GB)**

(54) **Method for accurate determining of CPR chest compression depth in real time**

(57) Method for accurately determining the real time CPR chest compression depth exercised on a patient by a performer by using an acceleration signal and a reference signal.

Fig. 1

EP 1 985 228 A2

**Description**

Introduction

The present invention relates to chest compression. More specifically, the invention describes a method to determine compression depth from acceleration and force.

Background of invention

CPR (Cardiopulmonary Resuscitation) feedback systems have recently gained attention as a method for improving the quality of CPR on a cardiac arrest victim. One typical feature of such systems is to measure the compression depth and rate during chest compressions, and compare these with accepted guideline limits and give verbal or visual feedback to the rescuer if, for instance the compression depth does not meet the accepted value of 3.8 - 5.1 cm.

A system for giving feedback on compressions typically consists of a sensor pad to be placed on the victim's chest. The sensor pad may contain an accelerometer and optionally a force sensor. The compression depth measurement is usually based on double integration of acceleration. However, if not all zero offset is removed from the acceleration signal prior to double integration, the integration is likely to "run off' and the estimated depth will not be useable for giving feedback.

US-6,306,107 to Myklebust et al describes a method for double integration to obtain chest compression depth, where the depth and velocity is reset to zero each time a force switch is activated at the onset of a new compression.

The publication "Compression Depth estimation for CPR Quality assessment Using DSP on Accelerometer Signals", IEEE Transactions on Biomechanical engineering, vol 49, no 3, Mar 2002, Aase et al describes a method where the offset in acceleration is removed after each compression, by setting the boundary conditions so that the chest is assumed to return to the same position and speed when the force is released. The integration limits determining the boundary conditions are found by help of a force switch. The disadvantage of this method is that the method does not provide a real-time, sample-by-sample assessment of depth, but only calculates depth of the previous compression.

US-7118542 to Palazzolo et al describes another technique for filtering and integrating acceleration to obtain depth. A moving average of past starting points is used to estimate the starting point of each compression. Additionally, an independent reference signal, such an ECG (Electrocardiogram) signal with compression artifacts, may be used to as a help to determine the starting points. Various types of noise reference signals may be used to estimate and remove sources of noise in the acceleration signal prior to integration, by correlating said noise reference signals with the acceleration signal.

One disadvantage with the method described in US7118542 is that it does not compensate the acceleration or depth signals for distortion caused by the filters. In general, filters do not only remove frequencies outside their pass-band, but also attenuates or delays certain frequency components within the pass-band. This may cause distortion of the filtered signal relative to the original version.

For instance, if a high-pass filter is used to remove drift in acceleration offset, the filter will also distort frequencies in the vicinity of its cutoff frequency. Upon double integration, these distortions will also cause distortion of the resulting depth signal. If the cut-off frequency of the filter is too close to the compression frequency, the depth signal may be significantly distorted. On the other hand, the lower the cut-off frequency, the less effective the filter will be in removing drift.

Filtering with a digital filter is a powerful and well-known technique for removing high frequency noise and/or drift from a digitized signal waveform. Digital filters can be tailored to different types (high-pass, low-pass, band-pass, band-stop), families (e.g. finite impulse response (FIR) or infinite impulse response (IIR)), and properties such as order, phase, ripple and cut-off/corner frequencies.

The aim of the current invention is to remove or reduce the effects of filter distortion of the compression depth signal, while maintaining an adequate removal of sensor drift.

In the proposed solution, this is accomplished by adding a compensation signal to the waveform obtained during filtering and integration. The compensation signal is found by filtering an independent reference signal with approximately the same, i.e. similar amplitude, shape and phase as the depth signal, using the same filters as have been used to remove offset drift in the acceleration. A compensation signal, equal to the difference between the raw and filtered reference signal, is then calculated and added to the compression depth signal to compensate for the distortion caused by the filter. This compensation signal is presumed to be approximately equal to the portion of the depth signal removed by the filter, thus giving a more accurate determination of the actual compression depth.

Summary of the invention

The present invention is a method for accurately determining real time chest compression depth x exercised on a patient or a manikin by a performer, by executing the following steps, on signals measured by a chest compression sensor unit used in the CPR exercise on the patient or manikin, at time t:

- receiving a signal $a(t)$ representing an acceleration value of the chest compression,

- applying one or more filters to the received acceleration signal *a(t)* for removing unwanted signal components,

- applying double integration on the signal after the said filtering step resulting in a raw depth signal *x_r(t),*

- using an independent reference signal R(t), changing it to produce an adjusted reference signal *r(t)* having similar amplitude, shape and phase as the raw depth signal *x_r(t),*

- obtaining a resulting compensation signal *x_c(t)* by subtracting a filtered adjusted reference signal *r_f(t)* from the adjusted reference signal *r(t),* by using the same filter(s) as applied to the received acceleration value *a(t),*

- determining the compression depth x at time *t* by adding the raw depth signal *x_r(t)* and the compensation signal *x_c(t).*

Other features of the inventive method are set forth in the enclosed dependent claims.
The invention further comprises a device for accurately determining real time chest compression depth x exercised on a patient or a manikin by a performer, characterized in that the device comprises a chest compression sensor unit used in the CPR exercise, and means for executing the method steps defined above.

Brief description of the drawings

In the following the invention will be explained in more detail, with reference to the accompanying drawings in which:

Fig. 1 shows a schematic description of the principle according to the invention;

Fig. 2a to 2i shows examples of waveforms illustrating various aspects of the invention, where

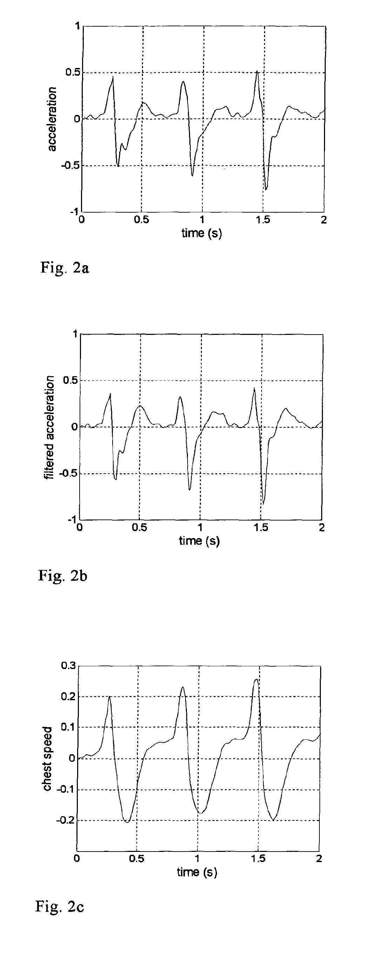

Fig. 2a shows measured acceleration,

Fig. 2b shows acceleration filtered by a digital comb filter,

Fig. 2c shows chest speed found by integrating filtered acceleration,

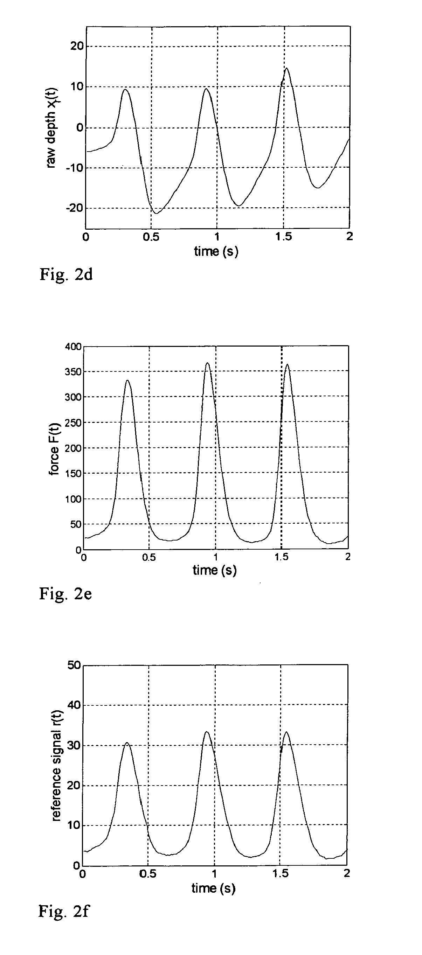

Fig. 2d shows raw depth found by integrating chest speed,

Fig. 2e shows compression force F(t) measured by force sensor,

Fig. 2f shows reference signal r(t) found by adjusting amplitude and phase of force signal F(t) to match depth signal as good as possible,

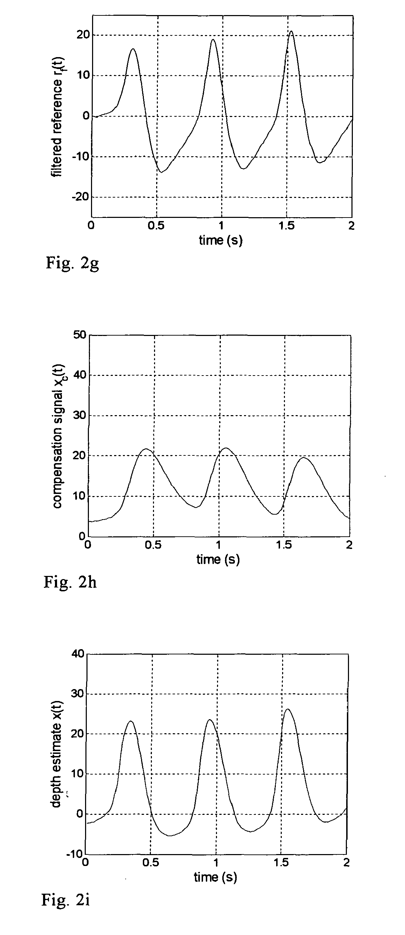

Fig. 2g shows *r_f(t),* the reference signal filtered using same filters as used to filter acceleration (ref. fig 2b),

Fig. 2h shows the compensation signal *x_c(t),* found by subtracting filtered reference signal *r_f(t)* from reference signal *r(t),* and

Fig. 2i shows *x(t),* the estimate of depth obtained by the proposed method.

Detailed description

According to the present invention, an accurate determination of the compression depth waveform x(t) as a function of time is given by the relation:

$$x(t) = x\_r(t) + x\_c\,(t),$$

where x_r(t) is a raw depth signal, and x_c(t) is a compensation signal.

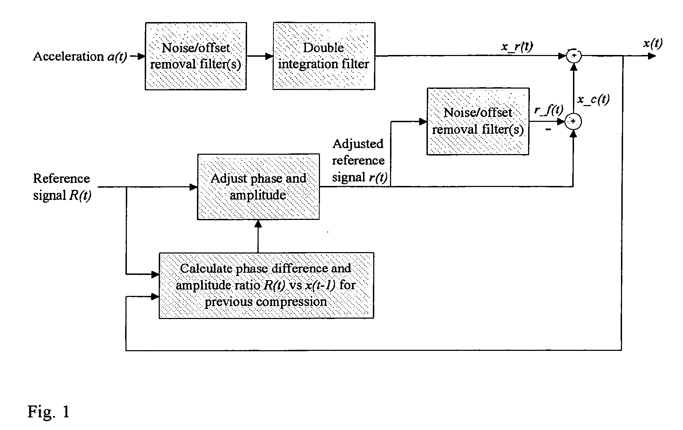

Fig. 1 shows a schematic description of the inventive method for accurately determining the real time chest compression depth x exercised on a patient or manikin by a CPR performer. The raw depth estimate x_r(t) (as shown in fig. 2d) is obtained by filtering and double integrating a received acceleration signal a(t). The acceleration a(t) is for instance measured by an accelerometer placed inside a CPR sensor unit that is applied on the sternum during compressions, and sampled at regular intervals.
Fig. 2a shows a typical waveform of measured acceleration.
To determine x_r(t), the measured acceleration signal is first filtered by one or several digital filters to remove unwanted signal components, e.g. offset drift and/or high frequency noise. One filter type which is particularly suited for this purpose is the so-called comb filter, which is a very simple infinite impulse response filter.
Fig. 2b shows the waveform of acceleration filtered by comb filter.
The filtered acceleration signal is then double integrated, for instance by use of an integrating digital filter. According to digital filtering theory, the order of the filters will not influence on the final result, and hence, the sequence of filtering and integration can be interchanged.
Fig. 2c shows the waveform of chest speed found by integrating the filtered acceleration, and Fig. 2d shows the waveform of raw depth signal x_r(t) found by integrating chest speed.
The compensation signal x_c(t) (waveform shown in fig. 2h) is obtained by subtracting a filtered reference signal r_f(t) (as shown in fig. 2g) from an original reference signal r(t) (as shown in fig. 2f). The original reference signal r(t) is produced from an independent reference signal R(t) by adjusting the signal R(t) to have similar amplitude, shape and phase as the raw depth signal x_r(t). The compensation signal x_c(t) thus represents the portion of the reference signal that has been removed by the filters.

In the process of filtering r(t) to obtain r_f(t), it is necessary that the employed filter(s) are the same as used to filter acceleration in the process of calculating x_r(t), or at least give essentially the same distortion of the signal.

In a preferred embodiment, the reference signal r(t) is deduced from measurements generated by a force sensor measuring compression force. The sensor, preferably situated between the palm of the hand of the rescuer and the victim's chest, samples force F(t), preferably synchronously with the accelerometer sampling. Other types of reference signals that can be used include ECG or thoracic impedance measurements with compression-related artifacts, or various kinds of blood pressure measurements. The independent reference signal R(t) may also comprise a combination of any information producing signals, for instance a combination of two or more of the signals mentioned above.

Fig. 2e shows the waveform of compression force F(t) measured by a force sensor.

Since the output signals from different types of sensors have different units and therefore in general different amplitudes, the numerical amplitude of the reference signal R(t) needs to be adjusted to essentially match the numerical amplitude of the depth signal x(t). The reference- and depth signals may also have a typical phase difference or delay which needs to be adjusted for.

This adjustment is produced by calculating the difference in phase and amplitude ratio of the independent reference signal R(t) and a previously determined compression depth signal x(t-1), ref. fig 1.

In the following the relation between the raw (measured) reference signal R(t), and the phase/amplitude adjusted reference signal r(t) will be further explained by an example.

Assuming use of compression force F(t) as reference signal gives R(t) = F(t). It is known that force is measured in Newton's and depth in mm. Thus the relationship between force amplitude in N, and depth amplitude in mm must be known. The ratio of force to depth during compressions will in the following be termed chest stiffness, k (N/mm). To calculate the reference signal r(t), the measured force F(t) must be divided by k, so that r(t)=F(t)/k.

Fig. 2f shows reference signal r(t) found by adjusting amplitude and phase of the force signal to match the compression depth signal as good as possible.

In addition, due to a viscous force component in the chest, depth usually is delayed in relation to force. Thus the phase of the amplitude-adjusted reference signal needs to be adjusted to better match with depth.

In one embodiment, the chest stiffness k is found by simply dividing the maximum force of previous compressions with the corresponding maximum depth. The depth employed in this calculation can for instance be the depth x(t) of previous compressions as estimated by the algorithm. The phase difference of the force and depth signals can be found by observing the difference in for instance maximum and minimum points of previous compressions force F(t) and depth x(t). For the first compression(s), a constant value of k and $\mu$ can be used in order to start the calculation. Depth can be absolute, i.e. measured relative to zero, or relative, i.e. measured relative to the previous point of lowest depth.

In a preferred embodiment, the amplitude and phase of the force signal is adjusted by assuming the following relationship between a measured compression force signal F(t) and an adjusted reference signal r(t):

$$F(t) = kr(t) + \mu v(t),$$

or

$$r(t) = (F(t) - \mu v(t))/k$$

v(t) is chest speed, which can for instance be determined by differentiating x(t) or alternatively r(t) with respect to time. k is chest stiffness and $\mu$ is termed chest damping. Both $\mu$ and k can be constants or variable with depth. The equation above can further be generalized to include acceleration.

For both the described embodiments, the stiffness k and damping component $\mu$, used to modify the amplitude and phase of the force signal, respectively, can be calculated as a function of depth from the waveforms of force and depth for previous compressions, for instance using the methods described in: "Compression force-depth relationship during out-of-hospital cardiopulmonary resuscitation" by Tomlinson A, Nysaether J, Kramer-Johansen J, Steen PA, Dorph E. Resuscitation, 2006 (in press), or "Anterior-Posterior Thoracic Force-Deflection Characteristics Measured During Cardiopulmonary Resuscitation": Comparison to Post-Mortem Human Subject Data Stapp Car Crash Journal", Vol. 50 by Kristy B. Arbogast, Matthew R. Maltese, Vinay M. Nadkarni, Petter Andreas Steen, Jon B. Nysaether,, November 2006, (in press).

The values for k and $\mu$ used in the calculation of r(t) can for instance be based on mean or median values of k and $\mu$ found for previous compressions.

The bottom points of the waveform x(t) may from time to time show significant offsets from zero. Thus, in general, the x(t) is more accurate in calculating the relative than the absolute compression depth of each compression. To accommodate for this, the depth output can be reset to zero after each compression, for instance at the point of minimum force or depth between two compressions.

Fig. 2i shows the estimate of depth x(t)obtained by the present inventive method.

Alternatively, in order to measure the absolute compression of the chest, the depth x(t) can be reset to a depth x0 after each compression, where x0 = F_min/k. Here F_min is the force at the point where depth is reset (Tomlinson et al, 2006).

It is observed that the signal r(t), having similar amplitude, shape and phase as the compression depth signal x(t), is in itself an estimate of compression depth. Under special circumstances, for instance during transport when acceleration is influenced by the movement of the patient backing surface, r(t) may give a better estimate of compression depth than x(t). Thus, when such situations are detected, the system may choose to display r(t) instead of x(t) as a representative of compression depth.

It may also be possible to make a hybridization of r(t) and x(t) and let this represent compression depth. For instance, one can let r(t) represent the shallowest part of the compression and x(t) the deepest part of the compression, and for instance let there be a gradual change from r(t) to x(t) in a predefined depth interval, for instance 10-20 mm depth."

In another application example, the sensor is used on a manikin for the purpose of calculating chest compression depth during CPR training. The sensor may be used externally on a manikin chest as if it were a real patient, or be integrated into the manikin.

**Claims**

1. Method for accurately determining real time chest compression depth x exercised on a patient or a manikin by a performer, **characterized in** executing the following steps, on signals measured by a chest compression sensor unit used in a CPR exercise, at time *t*:

   - receiving a signal *a(t)* representing an acceleration value of the chest compression,
   - applying one or more filters on the received acceleration signal *a(t)* for removing unwanted signal components,
   - applying double integration on the signal after said filtering step resulting in a raw depth signal *x_r(t)*,
   - using an independent reference signal R(t), changing it to produce an adjusted reference signal *r(t)* having similar amplitude, shape and phase as the raw depth signal *x_r(t)*,
   - obtaining a resulting compensation signal *x_c(t)* by subtracting a filtered adjusted reference signal *r_f(t)* from the adjusted reference signal *r(t),* by using the same filter(s) as applied to the received acceleration signal *a(t),*
   - determining the compression depth x at time t by adding the raw depth signal *x_r(t)* and the compensation signal *x_c(t).*

2. Method according to claim 1, **characterized in that** the filter is a comb filter.

3. Method according to claim 1, **characterized in that** the independent reference signal *R(t)* is deduced from a force signal *F(t)* generated by a force sensor measuring compression force exerted in the CPR exercise.

4. Method according to claim 1, **characterized in that** the independent reference signal *R(t)* is deduced from ECG signals.

5. Method according to claim 1, **characterized in that** the independent reference signal *R(t)* is deduced from thoracic impedance measurements.

6. Method according to claim 1, **characterized in that** the independent reference signal *R(t)* is deduced from blood pressure measurements.

7. Method according to claim 1, **characterized in that** the independent reference signal *R(t)* is deduced from a combination of any of the signals in claims 3, 4, 5 and 6.

8. Method according to claim 1, **characterized in that** the independent reference signal *R(t)* is deduced from a combination of a force signal *F(t)* and other measured information producing signals.

9. Method according to claim 1, **characterized in that** the adjusted reference signal *r(t)* is produced by calculating the difference in phase and amplitude ratio of the independent reference signal *R(t)* and a previously determined compression depth *x(t-1)* signal.

10. Method according to claim 1, **characterized in that** the accelerometer signal and the reference signal are synchronized.

11. Method according to claim 3, **characterized in that** the force sensor is situated between the palm of the hand of the performer and chest of the patient.

12. Device for accurately determining real time chest compression depth *x* exercised on a patient or a manikin by a performer, **characterized in that** the device comprises a chest compression sensor unit used in the CPR exercise, and means for executing the steps defined in claims 1 to 11.

Fig. 1

Fig. 2a

Fig. 2b

Fig. 2c

Fig. 2d

Fig. 2e

Fig. 2f

Fig. 2g

Fig. 2h

Fig. 2i

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6306107 B, Myklebust

- US 7118542 B, Palazzolo

**Non-patent literature cited in the description**

- Compression Depth estimation for CPR Quality assessment Using DSP on Accelerometer Signals. *IEEE Transactions on Biomechanical engineering,* March 2002, vol. 49 (3
- Compression force-depth relationship during out-of-hospital cardiopulmonary resuscitation. **TOMLINSON A ; NYSAETHER J ; KRAMER-JOHANSEN J ; STEEN PA ; DORPH E.** Resuscitation. 2006

- Anterior-Posterior Thoracic Force-Deflection Characteristics Measured During Cardiopulmonary Resuscitation. **KRISTY B. ARBOGAST ; MATTHEW R. MALTESE ; VINAY M. NADKARNI ; PETTER ANDREAS STEEN ; JON B. NYSAETHER.** Comparison to Post-Mortem Human Subject Data Stapp Car Crash Journal. November 2006, vol. 50